# EUROPÄISCHE PATENTSCHRIFT

(11) **EP 3 590 468 B1**
(45) Veröffentlichungstag und Bekanntmachung des Hinweises auf die Patenterteilung: **12.01.2022**
(21) Anmeldenummer: 19184044.6
(22) Anmeldetag: 03.07.2019
(51) Int. Cl.: A61D 9/00

(54) **KIEFER-TEMPERIERBAND FÜR NICHTMENSCHLICHE SÄUGETIERE NÄMLICH FÜR PFERDE ODER ANDERE REIT- UND ARBEITSTIERE**
JAW TEMPERING BELT FOR NON-HUMAN MAMMALS NAMELY FOR HORSES OR OTHER PORTAGE AND WORK ANIMALS
BANDE DE TEMPÉRAGE DE MÂCHOIRE POUR MAMMIFÈRES NON HUMAINS NOTAMMENT POUR CHEVAUX ET AUTRES ANIMAUX DE PORTAGE ET DE TRAVAIL

(30) Priorität: 06.07.2018 DE 202018103879 U
(43) Veröffentlichungstag der Anmeldung: 08.01.2020
(73) Patentinhaber: Sannemüller, Karin, 22589 Hamburg (DE)
(72) Erfinder: Sannemüller, Karin, 22589 Hamburg (DE)
(74) Vertreter: Eisenführ Speiser

(56) Entgegenhaltungen:
- DE-A1-102012 016 072
- US-A1- 2014 358 205

## Beschreibung

Die vorliegende Erfindung betrifft ein Kiefer-Temperierband für nichtmenschliche Säugetiere, insbesondere für Pferde. Gedacht ist aber auch an andere Reit- und Arbeitstiere, wie z.B. Esel, Kamel oder Maultier. Insbesondere ist daran gedacht, dass das Temperierband der gezielten Erwärmung bestimmter Kopfbereiche bzw. der gezielten Erwärmung von Muskeln im Kopfbereich des Säugetieres dienen soll. Insofern wird nachfolgend von einem Kiefer-Wärmeband gesprochen, abgekürzt KWB, ohne dass eine Beschränkung darauf beabsichtigt ist. Das besagte KWB ist nach ersten Erprobungen z.B. bei Pferden in der Lage, auf besonders einfache und wirkungsvolle Weise die Kiefer-/Kaumuskulatur des Tieres zu entspannen. Auch wenn nachfolgend vorwiegend die Anwendung beim Pferd beschrieben ist soll daran keine Beschränkung zu sehen sein.

Beim Menschen gibt es einen Symptomen-Komplex, der sich CMD (craniomandibuläre Dysfunktion) nennt. Das ist ein Oberbegriff für strukturelle, funktionelle, biochemische und psychische Fehlregulation der Muskel- oder Gelenkfunktion der Kiefergelenke. Diese Fehlregulation kann schmerzhaft sein. Die Deutsche Gesellschaft für Funktionsdiagnostik und Therapie definiert CMD als Sammelbegriff für eine Reihe klinischer Symptome der Kaumuskeln und/oder des Kiefergelenks sowie der dazugehörenden Strukturen im Mund-Kopfbereich.

Beim Pferd und artverwandten Tieren sind diese Strukturen ähnlich aufgebaut. Anders als beim Hund oder bei einer Katze wird beim Pferd in die natürliche Muskelspannung eingegriffen. Beim Menschen liegen zumeist Zivilisationsprobleme wie Stress oder psychosomatische Ursachen vor, beim Pferd ist es die falsche Haltung oder der falsche Beritt.

Beim Menschen können durch kräftiges Ziehen der Muskeln und Sehnen an den Schädelknochen Nervenirritationen sowie Schwindel, Zahnschmerzen ohne ersichtlichen Grund, Ohrenprobleme wie Tinnitus, Schwerhörigkeit sowie Ohrenschmerzen entstehen. Das Ohr ist beim Menschen genauso wie beim Pferd mit dem Kiefergelenk sowohl muskulär also auch nerval eng miteinander verknüpft.

Beim Pferd sind diese Muskelzüge und Schädelknochen ähnlich aufgebaut. Es ist nicht nachgewiesen, ob beim Pferd ähnliche Symptome auftreten. Doch jeder Reiter kennt das Problem, wenn das Pferd "nicht gut läuft" oder "auf einer Seite schlechter läuft" oder nicht am Nacken angefasst werden möchte oder das Gebiss nur widerwillig ins Maul nehmen möchte. Das sind alles Hinweise für starke Verspannungen mit Schmerzen. Genauso wie die Verkalkung des Nackenbandes und der daraus resultierenden Veränderungen. Das ist ein eindeutiges Zeichen für eine Überbeanspruchung der Sehnenansätze am Kopf. Wird an den Schädelknochen über verspannte Muskeln falsch gezogen, ändern sich die Spannungsverhältnisse der Schädelknochen, der Hirnhäute und deren umliegenden Strukturen und haben massive Einflüsse auf den Rest des Körpers.

Beim Pferd besteht die Kaumuskulatur aus den nachstehenden vier Muskelpaaren:
- Musculus masseter:: Führt den Unterkiefer an den Oberkiefer, um den Mund zu schließen, im Folgenden als Kaumuskel bezeichnet.
- Musculus temporalis:: Kieferschluss sowie Zurückziehen des Unterkiefers und minimales Vorschieben des Unterkiefers, im Folgenden als Schläfenmuskel bezeichnet.
- Musculus pterygoideus medialis:: Zuständig für den Kieferschluss, im Folgenden als innerer Flügelmuskel bezeichnet.
- Musculus pterygoideus lateralis:: Öffnen des Kiefers, Vorschieben des Unterkiefers. Zudem zuständig für die Mahlgleitbewegung, im Folgenden als äußerer Flügelmuskel bezeichnet.

Die Nacken-, Hals- und Kiefermuskeln arbeiten stets zusammen. Ist einer der drei verspannt, müssen die anderen mit anspannen. Aufgrund der Verspannung der Kaumuskeln ist zu erwarten, dass auch die Nacken- und die Halsmuskulatur des Pferdes sowie das Nackenband verspannt sind. Letzteres spielt für die Halsmuskulatur eine große Rolle, da es durch passive Haltearbeit den Muskeln Arbeit abnimmt.

Kaumuskeln und Kiefergelenke werden als eine funktionelle Einheit betrachtet. Das Kausystem leistet sehr komplexe, dauernde Gelenkbewegungen im Körper eines Pferdes. Vier Muskelpaare an Kiefer und Schläfen ermöglichen Dreh- und Seitenbewegungen (Mahlbewegungen) sowie das Vor- und Zurückziehen des Unterkiefers. Gleichzeitig bringen sie die größte punktuelle Kraft im Körper auf. Beschwerden und Anspannungen in diesem Bereich sind auf viele Ursachen zurückzuführen.

Ein Pferd, welches von Menschen gehalten und genutzt wird, ist immer wieder Stress-Situationen ausgesetzt.

Zu wenig Bewegung: Dauerndes Stehen ist für Pferde unangenehm. In der freien Natur ist ein Pferd bis zu 16 Stunden am Tag - meist zur Nahrungsaufnahme - in Bewegung und legt dabei viele Kilometer zurück. Von seiner Art her ist das Pferd ein Lauf- und Fluchttier. Dieser Bewegungsbedarf lässt sich allein über den Reitsport bzw. das Training nicht ausreichend abdecken.

Soziales Umfeld: Pferde sind ausgeprägte Herden- bzw. Gruppentiere. Jedes Pferd braucht die Möglichkeit, Sozialkontakte mit Artgenossen aufzunehmen. Bei jeder Haltungsform ist auf das soziale Gefüge und die Verträglichkeit der Pferde untereinander Rücksicht zu nehmen. Dies gilt auch für rasse-, alters- und geschlechtsspezifische Unterschiede.

Turniere: Pferde werden in der Turnierwelt meist zu früh geritten. Erst ab dem 5. bis 6. Lebensjahr schließen sich die letzten Wachstumsfugen und die Gelenke werden stabil. Jedoch werden viele Sportpferde bereits mit drei Jahren geritten, damit sie an den ersten Jungpferde-Prüfungen teilnehmen können. Dann müssen sie Anforderungen erfüllen, denen sie physisch und psychisch noch nicht gewachsen sind. Oft kommt es daher bei einem zu frühen Beritt zu Gelenkschäden, die nicht nur im betroffenen Gelenk, sondern auch im Kiefersystem ausgeglichen werden müssen. Eine Fehlstellung eines Gelenkes führt sich durch den Körper fort und macht sich somit auch im Kiefergelenk bemerkbar. Es kann zu Verspannungen und Dysfunktionen des Kiefergelenks und der umliegenden Strukturen kommen.

Unsachgemäßes Reiten und unpassendes Equipment: Stellt der Mensch das Pferd zu tief ein oder betreibt sogar die sogenannte Rollkur, bekommt das Pferd vermehrt Zug am Nackenband und dieses wird gereizt oder kann durch regelmäßigen zu starken Zug Kalk einlagern, um der Belastung standzuhalten. Dies belastet dann wiederum auch die umliegenden Strukturen.

Manche Reiter, gerade Anfänger, haben noch einen nicht ausbalancierten Sitz und das Pferd muss viel ausgleichen, was ebenfalls zu Verspannungen führen kann. Durch zu starken Zug am Gebiss kommt es teilweise zu Schmerzen in diesem Bereich und zu Abwehrreaktionen, welche wiederum Verspannungen auslösen.

Auch unpassendes Equipment kann Probleme im Kopf-Kiefer-System nach sich ziehen. Ein für das Pferdemaul zu großes oder breites Gebiss kann Schmerzen und Verspannungen in diesem Bereich hervorrufen. Ebenso eine unpassende Trense. Durch unsachgemäßen Gebrauch eines Kandaren-Zaums wird durch die Hebelwirkung Druck auf das Genick und so auch auf das Nackenband ausgeübt. Dies kann zu Schmerzen, Verspannungen und, nach regelmäßigem zu starkem Druck, auch zu Gewebeveränderungen führen.

Ein unpassender Sattel kann Rückenschmerzen und eine Dysbalance in der Rückenmuskulatur verursachen, welche sich bis in das Kiefergelenk fortsetzen. Die Kaumuskulatur muss diese Fehlspannung als letztes Körpergelenk ausgleichen. Sowohl die obere als auch die untere Halsmuskulatur hat direkten Kontakt zum Pferdekopf. Daher ist der Kopf mit seiner Kaumuskulatur der Dreh- und Angelpunkt jeglicher Befehle, die vom Reiter aus kommen und diese ausführen muss, egal, ob falsch oder richtig.

Falsche Zahnstellung: Ab ungefähr 2,5 Jahren beginnt der Zahnwechsel beim Pferd. Dabei kann es zu Problemen kommen, wie verzögertes Ablösen der Milchkappen, Schwellungen am Kiefer durch zu enge Zahnfächer und Schleimhaut- oder Zungenverletzungen durch scharfe Kanten an Zähnen und Milchkappen. Wenn sich die Zähne ungleich entwickeln, kann dies zu Problemen beim Kauen führen. Hieraus resultieren Verspannungen und Schmerzen im Kaumuskel-Apparat.

Zähne werden ein Pferdeleben lang durch dauerndes Mahlen abgerieben und schieben sich von unten nach. Der Zahn wächst nicht, die Wurzel wird kürzer. Geschieht dies nicht gleichmäßig, entstehen scharfe Haken, die vom Tierarzt korrigiert werden müssen. Bis dieses Problem erkannt wird, kaut das Pferd falsch und die Muskulatur verspannt. Dies löst meistens Schmerzen aus, da diese Haken die Mundschleimhaut verletzen können.

Viele Pferde haben sogenannte Wolfszähne. Diese sind evolutionäre Überbleibsel aus einer Zeit, in der die Pferde noch mehr Zähne besaßen als heute. Diese Zähne liegen dort, wo das Gebiss im Maul liegt. Dadurch kann es immer wieder zu Reizungen und Entzündungen des Zahnfleisches kommen. Die Wolfszähne können sich durch das Gebiss auch lockern und dadurch ebenfalls Entzündungen hervorrufen.

Ungepflegte, zu lange oder verschieden hohe Hufe: Die Hufe sind der Grundstein für eine gleichmäßige ausgewogene Haltung und für ein gleichmäßiges Gangbild des Pferdes. Sind sie asymmetrisch oder zu lang, wirken falsche Kräfte auf die Beine, in Verlängerung auf die Schultern und Hüften. Somit steht der Hals falsch und der Kopf muss diese Asymmetrie ausgleichen. Der Kiefer wird verspannt.

All dies führt zu verspannten Muskeln im Kopf/Kiefer-Bereich. Im Umkehrschluss gilt aber auch, dass eine entspannte Kaumuskulatur generell zur Entspannung von Körper und Geist beiträgt. Deswegen wirkt sich eine Entspannung der Kaumuskeln generell günstig aus, um Stress und Anspannung in der Muskulatur zu verringern.

In der US 2014/0358205 A1 werden Verfahren und Vorrichtungen beschrieben, um gezielt bestimmte Muskelbereiche eines Pferdes zu kühlen. Insbesondere ist dort beschrieben, wie bestimmte Muskelgruppen im Bereich eines Pferdekopfes mithilfe eines einteiligen Zuschnitts gekühlt werden können.

Die DE 10 2012 016 072 A1 beschreibt eine Bauch-oder Halsmanschette für Hunde, die mit Kammern ausgestattet ist, in die mit Heilmitteln gefüllte Säckchen eingelegt werden können.

Bisher ist nichts im Pferdebereich bekannt geworden, um z.B. gezielt die Kaumuskeln durch Wärme zu entspannen. Dies konnte bisher allenfalls erreicht werden, indem man Moorpackungen oder Kompressen oder Dinkel-, Traubenkern- oder Kirschkern-Kissen gegen die Muskeln hält. Das ist aber schwer durchführbar, da das Pferd stetig in Bewegung mit dem Kopf ist und beidseitig gehalten werden musste.

Es ist die Aufgabe der Erfindung, hier Abhilfe zu schaffen, also insbesondere ein Wärmeband für den Pferdekiefer bereitzustellen, das in der Lage ist, jedenfalls einige, nach Möglichkeit sogar die gesamten Kaumuskeln und die oberen Nackenmuskeln zu entspannen, wobei das Wärmeband zudem einfach zu handhaben und einfach zu verwenden sein soll.

Diese Aufgaben werden mittels eines Kiefer-Temperierbandes mit den Merkmalen des Anspruch 1 gelöst.

Vorteilhafte Aus- und Weiterbildungen, welche einzeln oder in Kombination miteinander eingesetzt werden können, sind Gegenstand der abhängigen Ansprüche. Die im Folgenden für Ausführungsformen des Kiefer-Temperierbandes genannten Vorteile sind gleichermaßen für eine Verwendung des Kiefer-Temperierbandes von Vorteil.

Gemäß der Erfindung sieht z.B. das als KWB ausgeführte Kiefer-Temperierband Aufnahmeeinrichtungen vor, die als Taschen ausgebildet sind und die zur Aufnahme von darin integrierbaren Energiespeichern eingerichtet sind, und das KWB soll zumindest teilweise den Unterkiefer, bevorzugt mit dem Zungenbein, den Oberkiefer, den Jochbeinbogen, das Keilbein sowie das Stirnbein und/oder das Scheitelbein des Pferdeschädels umspannen. Besonders bevorzugt ist das Umspannen aller dieser Bereiche, das Umspannen nur einzelner dieser Bereich führt aber auch bereits zu einer Muskelentspannung jedenfalls in diesem Bereich.

Die Aufnahmeeinrichtungen sind derart auf der z.B. dem Pferdekopf zugewandten Seite angeordnet, dass sie flächig und zumindest teilweise die Kaumuskeln, die Schläfenmuskeln, die inneren Flügelmuskel und die äußeren Flügelmuskeln und bevorzugt auch das Zungenbein abdecken. Dadurch lassen sich mit in diesen Aufnahmeeinrichtungen angeordneten Energiespeichern, z.B. mittels Wärmeträgern, in vorteilhafter Weise einzelne Muskeln oder Muskelgruppen der Kaumuskulatur gezielt erwärmen beziehungsweise entspannen.

Die als bevorzugt betrachtete und beabsichtige Erwärmung führt zu einer Erweiterung der Blutgefäße, die wiederum zu einer Entspannung der Muskulatur führt. Darüber hinaus macht sich eine schmerzlindernde Wirkung bemerkbar. Durch eine variable erfindungsgemäße Anordnung der Aufnahmeeinrichtungen kann über die Kiefermuskulatur hinaus auch ein Teil des Schläfenbereichs zwischen Augen und Ohren liegend mit einbezogen werden, sowie auch Muskulatur hinter den Ohren, das Nackenband und die kleinen Nackenmuskeln.

Bei einer vorteilhaften Ausführungsform der Erfindung sind die Aufnahmeeinrichtungen als Taschen in das Temperierband, insbesondere in das KWB eingenäht. Damit lässt sich das KWB sehr flach ausgestalten, wobei eine Bewegungsfreiheit des Kopfes auf einfache Art und Weise gewährleistet werden kann. Die Taschen können mehrere Kammern gleicher oder verschiedener Größen aufweisen. Dadurch ist es möglich, beliebige Energiespeicher gleicher oder verschiedener Größe zu verwenden.

Um der Größe der einzelnen Muskeln bzw. Muskelgruppen Rechnung tragen zu können, wird die Form und Größe der Aufnahmeeinrichtungen bevorzugt in Abhängigkeit einer vorgegebenen Muskelgröße gewählt. Dies erlaubt eine optimale Einstellung auf die Bedürfnisse eines betroffenen Pferdes. Anderseits ist eine Einheitsgröße der Aufnahmeeinrichtungen oder der darin vorgesehenen Kammern praktisch, wenn die Energiespeicher eine einheitliche Größe aufweisen.

Erfindungsgemäß ist das Kiefer-Temperierband mehrteilig ausgeführt. Dies erleichtert insbesondere das Anlegen des Bandes an den Kopf des Säugetieres. Je nach Tier erweist sich dieses Anlegen nämlich als durchaus problematisch, z.B. wenn das Tier scheu ist oder ein Manipulieren im Kopfbereich ungewohnt ist. Erfindungsgemäß ist dabei eine Unterteilung in ein Stirnband und in ein Nacken-Unterkieferband.

Es ist weiterhin bevorzugt, dass das Nacken-Unterkieferband mit bis zu acht gleich großen eingenähten Taschen versehen ist, um eine besonders leichte und verwechslungsfreie Handhabung zu ermöglichen. Dadurch können z. B. Wärmeträger als Energiespeicher praktisch in jede Tasche und in jeder beliebigen Lage gesteckt werden. Der Nutzer muss nicht darauf achten, welcher Energieträger wie herum in welche Tasche eingeführt werden muss. Abweichend sind auch mehr oder weniger als acht Taschen möglich. Die später beschriebene Ausführungsform besitzt acht Taschen, passend für Wärmeträger in der Größe von 11 x 19 Zentimeter. Je nach Pferdekopfgröße, beispielsweise Ponys, eignen sich auch Wärmeträger kleiner der angegebenen Größe. Auch größere sind möglich. Die Bandbreite reicht von 8 x 16 Zentimeter bis hin zu 20 x 30 Zentimeter. Energiespeicher z.B. mit quadratischer Grundform wären besonders leicht in die Taschen applizierbar, da in jeder Ausrichtung passend.

Das Nacken-Unterkieferwärmeband 1 weist erfindungsgemäß an seinen voneinander abgewandten unteren Enden Verbindungsmittel auf, z.B. bevorzugt zueinander komplementär angeordnete und ausgebildete Klettstreifen, um eine feste und einfache Verbindung zu garantieren. Da Klettbänder bekanntermaßen aus zwei Teilen bestehen, sind eine Befestigungsseite auf dem KWB mit einem Häkchenband und der Gegenpart mit einem komplementär angeordneten Flauschband versehen. Auf diese Weise lässt sich das KWB passgenau am Kopf des Pferdes anbringen und wieder lösen. Die Verbindung befindet sich bevorzugt unterhalb des Unterkiefers. Als Verbindungsmittel können aber auch Haken und Ösen oder Druckknöpfe oder Knotenbänder oder Gurt und Schnalle oder andere Verbindungsmittel dienen.

Vorteilhafterweise weist das Nacken-Unterkieferband 1 an seiner oberen linken Ecke ein rechtwinklig abgehendes elastisches Band auf, an dessen Ende sich ein Klettstreifen befindet. Das elastische Band spannt über Oberkiefer und Nasenbein des Pferdes und garantiert eine feste, passgenaue und einfache Verbindung durch einen komplementär angeordneten Klettstreifen an der oberen rechten Ecke. Die Verbindung liegt bevorzugt an der Wange unterhalb des Auges.

Für eine bessere Passform werden an der den Ohren zugewandten Seite des Nacken-Unterkieferbandes 1 zwei muldenförmige Aussparungen vorgesehen, wodurch sich das Pferd an den empfindlichen Ohren nicht eingeengt fühlt.

Es ist natürlich auch denkbar und im Umfang der Erfindung enthalten, die Verbindungsmittel als Haken mit Öse oder als Druckknopf oder als Gurt mit Schnalle oder als Klickverschluss oder als Band mit Schleife auszubilden.

Zum vorstehend erfindungsgemäß als zweiteilig ausgebildet beschriebenen KWB gehört ein zweites, separat anzubringendes, sogenanntes Stirnband 2. Es wird z.B. mittels Klettverschluss mit dem Nacken-Unterkieferband 1 verbunden und spannt sich oberhalb der Augenbrauen und unterhalb der Ohren quer über die Stirn des Pferdes. Der an den Enden schräge Grundschnitt (Trapezform) und die dort vernähten Klettbänder erlauben, das Stirn-Kieferwärmeband längenvariabel anzubringen und so optimal der Größe und der Anatomie des Pferdekopfes anzupassen.

Es ist natürlich auch denkbar und im Umfang der Erfindung enthalten, als Verbindungsmittel des Stirnbandes Haken mit Öse oder Druckknopf oder Gurt mit Schnalle oder Klickverschluss oder Band mit Schleife zu verwenden.

Das Stirnband 2 ist wie das Nacken-Unterkieferband 1 mit Aufnahmeeinrichtungen für Energiespeicher versehen, um beim Pferd gezielt die Muskulatur im Bereich des Kiefergelenks z.B. mit Wärme zu versehen und eine Entspannung herbeizuführen. Alternativ können anstelle der Energiespeicher wie Gel-Pads, Getreide-Kissen und Kirschkern-Kissen auch ein wärmeisolierender und/oder wärmereflektierender und/oder thermischer Stoff eingesetzt werden. Diese aufgeführten Stoffe sind Bestandteil der Erfindung.

Bei einer besonders bevorzugten Ausführungsform weist das KWB zweckmäßig zumindest einen elastischen Materialanteil auf. Dieser kann entweder bereits in dem Material des Wärmebandes verwebt sein oder in unterschiedlichen Abschnitten des Wärmebandes angeordnet /eingebracht sein. Der elastische Materialanteil erlaubt es, über das gesamte KWB hinweg einen leichten Anpressdruck aufzubauen, der ein vorteilhaftes Anschmiegen der mit Energiespeichern gefüllten Aufnahmeeinrichtungen gestattet und damit eine optimale Druckverteilung und Wärmeenergie-Übertragung erlaubt.

Bei einer bevorzugten Ausführungsform umfasst das Material des KWB eine Mischung aus Baumwolle und Polyurethan. Ein solches Gewebe ist beispielsweise das unter dem Markennamen Elasthan bekannte und Polyurethan enthaltene Material. Es hat sich gezeigt, dass diese Materialmischung besonders widerstandsfähig und leicht zu reinigen ist.

Bei einer anderen bevorzugten Ausführungsform umfasst das Material des Kiefer-Wärmebandes eine Mischung aus Baumwolle und Nylon oder aus Nylon/Polyester oder ausschließlich Nylon. Es hat sich gezeigt, dass diese Materialmischung oder pures Nylon besonders widerstandsfähig, glattflächig und wasserabweisend und leicht zu reinigen ist. Zudem können die Pferdehaare nicht so leicht an dem Material hängen bleiben. Durch die glatte Oberflächenstruktur des Materials lassen sich zudem die Energiespeicher leichter in die Aufnahmeeinrichtungen einführen.

Bei einer weiteren Ausführungsform umfasst das Material des Kiefer-Wärmebandes einen wärmeisolierenden und/oder wärmereflektierenden und/oder thermischen Stoff, ohne dass dafür Energiespeicher in Behältnissen verwendet werden müssen. Auch damit wäre eine entspannende Wirkung auf die gesamte oder/und teilweise Kaumuskulatur des Pferdes erreichbar.

Bei jeder Ausführungsform sind die beiden Wärmebänder bevorzugt derart gestaltet, dass der äußere Stoff eine isolierende Wirkung nach außen besitzt, wohingegen der innere Stoff, jener zwischen Energiespeichen, z.B. einem Wärmekissen, und Haut des Pferdes, eine gute wärmeübertragende Wirkung besitzt.

Gemäß einer weiteren Ausführungsform ist das KWB an eine vorgegebene Größe des Schädels angepasst. Um einen optimalen Einsatz zu gewährleisten, kann das Kiefer-Wärmeband in unterschiedlichen Größen ausgeführt werden, damit es eine optimale Passform und einwandfreien Tragekomfort beim Gebrauch gewährleistet.

Bei einer bevorzugten Ausführungsform der Erfindung sind die Energiespeicher in Form eines Kissens mit zumindest einer Kammer ausgebildet. Dadurch können die Energiespeicher sehr flexibel zusammen mit dem KWB eingesetzt werden, wobei sie in ergonomischer Weise aufgrund ihrer flachen Ausführung die Bewegungsfreiheit des Schädels kaum einschränken und damit wiederum den Tragekomfort erhöhen. Mit Energiespeicher sind bevorzugt Wärmespeicher gemeint.

Um eine vielseitige Einsatzmöglichkeit zu gewährleisten, sind die Energiespeicher mit einem handelsüblichen Gel oder einem Naturprodukt gefüllt. Als vorteilhaft lassen sich dabei auch Dinkelkörner oder Rapssamen oder Reis oder Traubenkerne oder Kirschkerne als Füllmittel für den Energiespeicher verwenden, sowie Mischungen daraus. Dabei wird in vorteilhafter Weise auch einer späteren ökologisch vertretbaren/verträglichen Entsorgung der Energiespeicher Rechnung getragen.

Bei einer bevorzugten Ausführungsform der Erfindung bestehen die Energiespeicher aus elektrisch beheizbaren Kissen (Pads) oder aus nur einmal zu verwendenden Wärme-Pads, deren Zellen z.B. Eisen, Aktivkohle, Salz und Wasser enthalten. Bei einer weiteren Ausführungsform der Erfindung enthalten die Energiespeicher einen oder mehrere Magneten.

Ein weiterer Aspekt der Erfindung betrifft die Verwendung der zuvor beschriebenen Kiefer-Temperierbänder, wobei die in/an dem Kopf umspannenden KWB vorgesehenen Aufnahmeeinrichtungen derart angeordnet und mit z. B. wärmenden Energiespeichern gefüllt sind, dass das KWB über eine dem Kopf zugewandte und damit den Aufnahmeeinrichtungen gemeinsame Fläche gleichzeitig an die Kaumuskel und/oder die Schläfenmuskeln und/oder die inneren und/oder äußeren Flügelmuskeln grenzt und diese erwärmt. Dadurch kann in besonders wirksamer Weise die Kaumuskulatur teilweise, bevorzugt insgesamt entspannt und damit ein besonders vorteilhafter psychosomatischer Entspannungseffekt erzeugt werden.

Darüber hinaus eignet sich die Art der Verwendung der Kiefer-Temperierbänder insbesondere vor einer Zahnarztbehandlung, bei der das Pferd länger den Mund offen halten muss. Dadurch können Folgeschmerzen und Spannungen vermieden werden, die bei vielen Pferden wegen einer Überlastung ihrer angespannten Kiefermuskulatur während der Behandlung auftritt.

Wie bereits eingangs erwähnt kann eine Entspannung der Kiefermuskulatur auch vorbeugend bei Nackenschmerzen sein. Dadurch kann das gesamte Wohlbefinden des Pferdes erheblich verbessert werden. Darüber hinaus ist der Einsatz des KWB einfach zu handhaben und mit keiner Gefahr verbunden.

Weitere vorteilhafte Ausgestaltungen der vorliegenden Erfindung werden nachfolgend bezugnehmend auf zwei Figuren näher erläutert, die zwei Ausführungsbeispiele darstellen. Dabei zeigen die Figuren jeweils eine skizzenhafte Darstellung eines Pferdekiefer-Wärmebandes gemäß vorteilhafter Ausführungsformen.

Anhand der beiden Darstellungen soll der Aufbau und die Anordnung und die sich daraus ergebende Funktionalität eines Pferdekiefer-Wärmebandes gemäß vorteilhafter Ausführungsbeispiele erläutert werden.

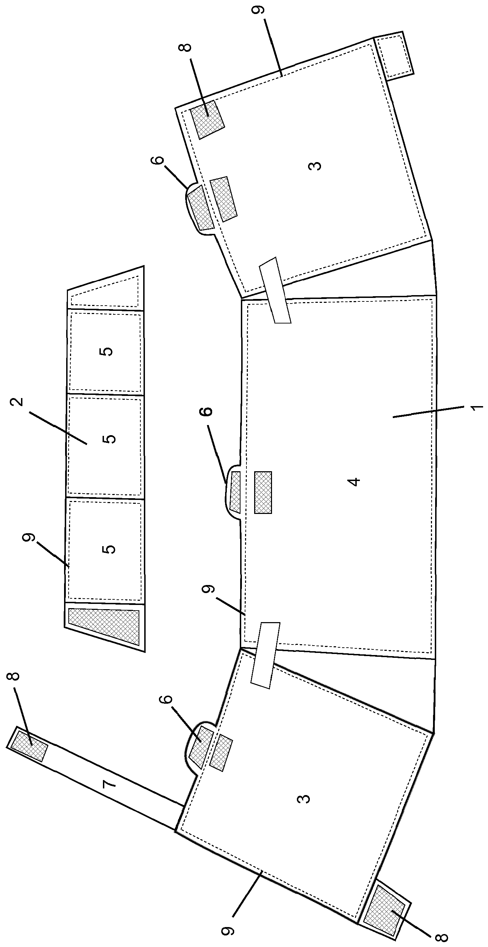

Das in Figur 1 dargestellte KWB, bestehend aus einem Nacken-Unterkieferwärmeband 1 und einem Stirn-Kieferwärmeband 2, ist dazu ausgelegt, den Kopf des Pferdes einmal vom Nacken (Atlas) beidseitig hinunter zum linken und rechten Unterkiefer und einmal quer über die Stirn vom linken zum rechten Kiefergelenk zu umspannen.

Beide, das Nacken-Unterkieferwärmeband 1 und das Stirn-Kieferwärmeband 2 weisen Taschen 3, 4 und 5 auf, in die Energiespeicher 9 eingebracht werden können. Diese Taschen sind ein Beispiel für Aufnahmeeinrichtungen und stehend stellvertretend für andere Mittel, um Energiespeicher im oder am Kiefer-Temperierband und somit am Kopf eines Säugetieres zu positionieren. In der Figur sind die Taschen 3, 4 und 5 durch gestrichelte Linien angedeutet. Die Energiespeicher weisen die Form eines flachen Kissens auf - hier nicht gezeigt. Diesbezüglich können die Taschen 3, 4 und 5 beispielsweise aus einer ersten Material-, beziehungsweise Stofflage, die dem Fell des Pferdekopfes zugewandt ist und einer zweiten oberen Material-, beziehungsweise Stofflage hergestellt werden, die an der von dem Fell des Pferdes abgewandten Seite diese erste Material-, beziehungsweise Stofflage überlagert. Die übereinander angeordneten Material-, beziehungsweise Stofflagen werden mittels Quernähten und Saumnähten entlang ihres äußeren Umfangs und miteinander verbunden, wobei dadurch die Taschen 3, 4 und 5 ausgebildet werden - vergleiche gestrichelte Linien. In diesen Taschen 3, 4 und 5 können dann die Energiespeicher 9 eingeführt werden.

Vorzugsweise werden die Energiespeicher 9 jeweils auf der Außenseite über Öffnungen eingebracht. Die Energiespeicher 9 sind bevorzugt Wärmespeicher. Bei einer weiteren dienlichen Ausführungsform können die Energiespeicher 9 jedoch auch als Kaltspeicher (Cool-Pads) ausgebildet sein und eingesetzt werden. Dadurch lassen sich das mit Energiespeichern 9 bestückte Nacken-Unterkieferwärmeband 1 und das Stirn-Kiefernwärmeband 2 noch flexibler und vielseitiger einsetzen und an unterschiedliche Verwendungen optimal anpassen.

Der Zweck des Nacken-Unterkieferwärmeband 1 und das Stirn-Kiefernwärmeband 2 ist es, die Kaumuskulatur eines nichtmenschlichen Säugetieres, bevorzugt die eines Pferdes, aber auch die Kaumuskulatur anderer Reit- und Arbeitstiere zu erwärmen und zu entspannen. Um z.B. die gesamte Kaumuskulatur erwärmen zu können, muss das Kiefer-Wärmeband 1 und 2 überall dort Taschen 3, 4 und 5 zur Aufnahme von Energiespeichern 9 aufweisen, wo eine Berührungsfläche des Kopfes, bzw. der Kaumuskulatur mit einer dem Kopf zugewandten Seite des Kiefer-Wärmebandes 3, 4 und 5 in Berührung kommt. Wenn lediglich eine teilweise Erwärmung der Kaumuskulatur erreicht werden soll wären lediglich in einem zugeordneten Bereich Aufnahmeeinrichtungen für Energiespeicher vorzusehen.

Im vorliegenden Ausführungsbeispiel sind die Taschen 3, 4 und 5 derart ausgebildet, dass die von dem Kiefer-Wärmeband 1 und 2 mit Energiespeichern 9 in Berührung gebrachten Kopfflächen einen Bereich der Kaumuskel, Schläfenmuskel, inneren Flügelmuskel und äußeren Flügelmuskel umfasst. Das in der Figur 1 dargestellte Nacken-Unterkieferwärmeband 1 und das Stirn-Kieferwärmeband 2 ist symmetrisch ausgeführt, so dass der gesamte Kiefer von dem Nacken-Unterkieferwärmeband 1 und das Stirn-Kiefernwärmeband 2 abgedeckt wird. In dem vorliegenden Ausführungsbeispiel sind die Aufnahmeeinrichtungen 3, 4 und 5 fest in dem Nacken-Unterkieferwärmeband 1 und in das Stirn-Kiefernwärmeband 2 eingenäht - gestrichelte Linien in der Figur deuten die Nähte beziehungsweise einen Saum an. Die Taschen 3 und 4 sind mit einem beide Stofflagen überlappenden Verschluss versehen, vorzugsweise als schnell zu öffnenden und schließenden Klettverschluss (Häkchen/Flausch), um ein Herausrutschen der Energieträger 9 zu verhindern.

Bei weiteren Ausführungsvarianten des Nacken-Unterkieferwärmebands 1 und das Stirn-Kiefernwärmebands 2 sind die Verschlüsse 6 als Reißverschluss, als Gurt/Schnalle, als Nietverschluss, als Knopfverschluss oder als Blitz- oder Klickverschluss oder als Band/Schlaufe ausgebildet.

Das dargestellte Nacken-Unterkieferwärmeband 1 ist symmetrisch aufgebaut und knickt zu seinen Enden in einem Winkel von zirka 30 Grad nach oben ab. Hiermit werden eine bessere Passform und ein höherer Tragekomfort gewährleistet. Die mittlere Tasche 4 ist als Rechteck (mit einem Maß z.B. zwischen 20 x 10 und 40 x 30 Zentimeter, hier ausgeführt mit 36 x 18 Zentimeter) ausgebildet, die beiden äußeren Taschen 3 ( mit einem Maß z.B. zwischen 15 x 15 und 30 x 30 Zentimeter, hier ausgeführt mit 23 x 23 Zentimeter) haben eine quadratische Form. In weiteren Ausführungsvarianten sind die Aufnahmeeinrichtungen 3 und 4 in runder, ovaler, elliptischer Form oder als Pentagon, Hexagon oder Oktagon ausgebildet.

Das dargestellte Nacken-Unterkieferwärmeband 1 ist an seinem linken oberen Ende rechtwinklig mit einem elastischen Verbindungsmittel, vorzugsweise Stretch-Band, versehen, an dessen Ende sich ein Verschluss, vorzugsweise Klettverschluss, Nietverschluss, Gurt/Schnalle-Verschluss oder Knopfverschluss befindet, um das Kiefer-Wärmeband an den Wangen des Pferdes zu fixieren. Das elastische Verbindungsband verläuft hierzu quer über das Nasenbein des Pferdes. Im vorliegenden Ausführungsbeispiel ist das Verbindungsmittel 7 als komplementär angeordneter Klettverschluss 8 ausgebildet. Der Vorteil dieses Kletterschlusses 8 ist, dass dieser eine passgenaue Verbindung zweier Enden des Nacken-Unterkieferwärmebandes 1 auf der Schädeldecke ermöglicht. Dadurch kann ein optimaler Sitz des Nacken-Unterkieferwärmebandes 1 gewährleistet werden.

Das dargestellte Stirn-Kieferwärmband 2 ist symmetrisch, hat einen länglich-trapezförmigen Zuschnitt und weist im vorliegenden Ausführungsbeispiel eine Größe von z.B. 45 x 8 cm auf. Größen von 30 bis 60 cm (Länge) und Höhen von 6 bis 12 cm sind ebenfalls möglich und Bestandteil dieser Erfindung. Unterteilt ist das Stirn-Kieferwärmband 2 in drei durch Nähte abgesteppte und gleich große Kammern, die zur Aufnahme von Energiespeichern/Wärmeträgern dienen. Die Kammern/Taschen sind nach oben hin offen, um ein leichtes Einstecken der Wärmeträger zu gewährleisten. Die Kammern bei einer Drei-Kammer-Lösung besitzen eine Größe von 7 x 9 Zentimeter. Abweichend davon kann das Stirn-Kieferwärmband 2 aber auch als ZweiKammer-Band ausgeführt werden (z.B. als kleinere Ausführung für Ponys). Ebenso ist eine Lösung mit nur einer länglichen Kammer über das gesamte Stirn-Kieferwärmeband 2 möglich, die zur Aufnahme eines in Kammergröße hergestellten Energiespeichers/Wärmeträgers dient.

An den Enden des Stirn-Kieferwärmebandes sind als Verbindungsmittel komplementär angeordnete Klettverschlüsse eingenäht, um mit dem Nacken-Unterkieferwärmeband 1 eine passgenaue und optimale Verbindung zu einzugehen. Die schräge Anordnung der Klettverschlüsse erlaubt zudem eine individuelle und optimale Anpassung an die Größe und an die Anatomie des Pferdekopfes. Das Stirn-Kieferwärmeband 2 spannt sich quer über die Stirn des Pferdes und sitzt oberhalb der Augenbrauen und unterhalb der Ohren. Dadurch wird gezielt ein Wärmeeintrag auf das Kiefergelenk und die das Gelenk umgebenden Muskeln des Pferdes erreicht.

In weiteren Ausführungsvarianten kann statt des Klettverschlusses auch ein Knopfverschluss, Nietverschluss oder eine Verbindung über Gurt/Schnalle oder Band/Schlaufe oder Klickverschluss mit elastisch ausgeführten Riemen vorgesehen sein.

Bei allen Ausführungsvarianten der Verbindungsmittel 7 und 8 ist gewünscht, dass sie einfach benutzt werden können.

Bei dem in der Figur dargestellten Ausführungsbeispiel können die Energiespeicher 9 in sämtliche Taschen 3, 4 und 5 eingebracht werden, wobei dadurch eine Entspannung der gesamten Kaumuskulatur bewirkt werden kann. Eine Einbringung von Energiespeichern 9 kann auch nur punktuell erfolgen, wenn lediglich einzelne Bereiche der Kaumuskulatur erwärmt/behandelt werden sollen. Die Energiespeicher 9 sind mit Vorteil mit einem Gel oder einem Naturprodukt gefüllt. Bei einer besonders bevorzugten Ausführung werden als Naturprodukt Dinkelkörner oder Rapssamen oder Reis oder Trauben- oder Kirschkerne verwendet. Diese Naturprodukte sind nicht nur hautverträglich, sondern auch ökologisch abbaubar und haben zudem eine geringes Gewicht, was den Tragekomfort weiter erhöht.

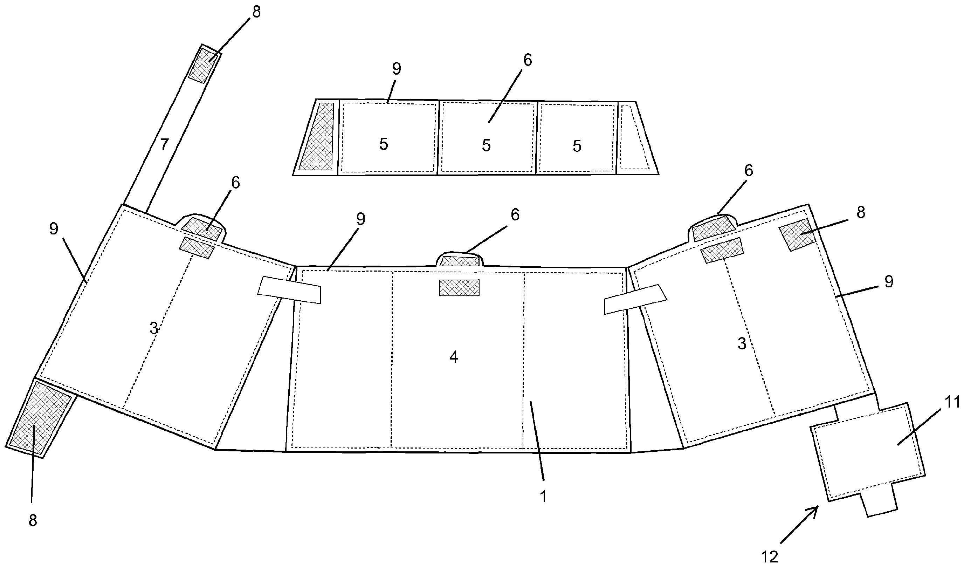

Das in Figur 2 dargestellte KWB ist in weiten Teilen zu dem in Fig. 1 gezeigten WB identisch. Es werden nachfolgend lediglich die Unterschiede beschrieben.

Im Ausführungsbeispiel der Fig. 2 ist vorgesehen, dass die Taschen 3, 4 und 5 des Stirn-Kieferwärmebandes noch einmal in Untertaschen unterteilt sind. So sind z.B. die beiden seitlichen Taschen 3 in zwei gleich große Subtaschen unterteilt. Die zentrale Tasche 4 ist in drei Subtaschen unterteilt. In diese genannten Subtaschen kann jeweils ein Energiespeicher 9 eingebracht werden. Wenn andere Aufnahmeeinrichtungen als Mittel vorgesehen sind, um Energiespeicher im oder am Kiefer-Temperierband und somit am Kopf eines Säugetieres zu positionieren, können auch diese anderen Aufnahmeeinrichtungen entsprechend noch einmal unterteilt sein.

Als weiterer Unterschied zwischen den Ausführungsbeispielen der Figuren 1 und 2 ist auf ein Wärmekissen 11 für das Zungenbein hinzuweisen. Hierzu ist der zu Figur 1 beschriebene Klettverschluss 8 länger ausgeführt und er wirkt mit einer Zungenbein-Tasche 12 zusammen, die in ihrer Dimensionierung bevorzugt so gewählt wurde, dass die Kissen für die Stirn-Taschen 5 auch in diese Zungenbein-Taschen 12 eingesetzt werden können

Die vorliegende Erfindung ermöglicht es, auf besonders einfache und wirkungsvolle Weise die Kiefermuskulatur des Pferdes zu entspannen, wobei dadurch auch Nackenschmerzen/-verspannungen vorgebeugt werden können. Insgesamt kann das Wohlbefinden des Tieres erheblich verbessert werden.

## Patentansprüche

1. Kiefer-Temperierband (1, 2) für nichtmenschliche Säugetiere, nämlich für Pferde oder andere Reit- und Arbeitstiere wie Esel, Kamel und Maultier, umfassend einen der Geometrie des Pferdes oder anderen Reit- und Arbeitstieres angepassten Grundzuschnitt, wobei der Grundzuschnitt Aufnahmeeinrichtungen (3, 4 , 5) aufweist zur Aufnahme von wärmenden Energiespeichern(9), und wobei das Kiefer-Temperierband (1, 2) zumindest teilweise den Unterkiefer, bevorzugt mit Zungenbein, den Oberkiefer, den Jochbeinbogen, das Keilbein, das Stirnbein und/oder das Scheitelbein des Kopfes und/oder den Nacken des Pferdes oder anderen Reit- und Arbeitstieres umspannt, wobei zumindest eine Aufnahmeeinrichtung (3, 4, 5) in/an dem den Kopf umspannenden Kiefer-Temperierband (1, 2) angeordnet ist, und das Kiefer-Temperierband (1, 2) über eine dem Kopf zugewandte und mit der Aufnahmeeinrichtung (3, 4, 5) gemeinsame Fläche an einen Kaumuskel und/oder einen Schläfenmuskel und/oder einen inneren Flügelmuskel und/oder einen äußeren Flügelmuskel des Schädels grenzt, wobei die Aufnahmeeinrichtungen als Taschen (3, 4, 5) ausgebildet sind, wobei der Grundzuschnitt wenigstens zweiteilig ausgebildet ist und aus wenigstens zwei Bandelementen (1, 2) besteht, wobei an den Bandelementen Verbindungsmittel (7, 8) angeordnet sind zum lösbaren Verbinden der Bandelemente, wobei ein Bandelement, nämlich ein Stirnband (2), als über die Säugetierstirn verlaufend, und ein anderes Bandelement, nämlich ein Nacken-Unterkieferband (1), als über den Säugetierunterkiefer und/oder über den Säugetiernacken verlaufend ausgeführt ist, wobei das Nacken-Unterkieferband (1) zumindest teilweise den Nacken, den Unterkiefer, bevorzugt mit Zungenbein, den Oberkiefer und den Jochbeinbogen umspannt, und wobei das Stirnband (2) zumindest teilweise das Keilbein, das Stirnbein und das Kiefergelenk des Tieres umspannt, wobei das Nacken-Unterkieferband (1) an seinen voneinander abgewandten Enden ein Verbindungsmittel (8) aufweist, mit dem die beiden Enden verbindbar sind, **dadurch gekennzeichnet, dass** das Stirnband (2) an seinen Enden ein Verbindungsmittel (7, 8) sowie das Nacken-Kieferband (1) zugehörige Verbindungsmittel (7, 8) aufweist, so dass die beiden Bänder verbindbar sind.

2. Kiefer-Temperierband (1, 2) nach Anspruch 1, **dadurch gekennzeichnet, dass** die Aufnahmeeinrichtungen (3, 4, 5) als Taschen fest in dem Nacken-Unterkieferband (1) sowie in dem Stirnband (2) integriert sind, wobei die Aufnahmeeinrichtungen (3, 4, 5) jeweils bevorzugt in mehrere Taschen unterteilt ausgebildet sind.

3. Kiefer-Temperierband (1, 2) nach einem der vorhergehenden Ansprüche, **dadurch gekennzeichnet, dass** die Aufnahmeeinrichtungen (3, 4, 5) mittels eines Befestigungsmittels (10) an das Nacken-Unterkieferband (1) sowie an dem Stirnband (2) fixierbar sind.

4. Kiefer-Temperierband (1, 2) nach einem der vorhergehenden Ansprüche, **dadurch gekennzeichnet, dass** eine Form und Größe der Aufnahmeeinrichtung (3, 4, 5) in Abhängigkeit einer vorgegebenen Muskelgröße wählbar ist.

5. Kiefer-Temperierband (1, 2) nach einem der vorhergehenden Ansprüche, **dadurch gekennzeichnet, dass** das Verbindungsmittel (7, 8) als ein Klettverschluss oder als ein Nietverschluss oder als ein Gurt/Schnalle-Verschluss oder als ein Klickverschluss oder als ein Band/Schlaufen-Verschluss ausgeführt ist.

6. Kiefer-Temperierband (1, 2) nach einem der vorhergehenden Ansprüche, **dadurch gekennzeichnet, dass** es mindestens einen elastischen Materialanteil aufweist, insbesondere wobei das Nacken-Unterkieferband (1) und das Stirnband (2) mindestens einen elastischen Materialanteil aufweist, wobei das Material des Kiefer-Temperierbandes (1, 2), insbesondere des Nacken-Unterkieferbandes (1) und/oder des Stirnbandes (2), bevorzugt aus einer Mischung aus Baumwolle und Nylon oder aus einer Mischung aus Nylon und Polyester oder ausschließlich aus Nylon besteht.

7. Kiefer-Temperierband (1, 2) nach einem der vorhergehenden Ansprüche 1 bis 6, **dadurch gekennzeichnet, dass** das Nacken-Unterkieferband (1) und/oder das Stirnband (2) an eine vorgegebene Größe des Säugetierkopfes angepasst ist.

8. Kiefer-Temperierband (1, 2) nach einem der vorhergehenden Ansprüche, **dadurch gekennzeichnet, dass** das Nacken-Unterkieferband (1) ein elastisches Fixierband (7) aufweist, dessen Ende eine Befestigung besitzt, die sich zwischen Nasenbein und Wannenknochen des Säugetieres befindet.

9. Kiefer-Temperierband (1, 2) nach einem der vorhergehenden Ansprüche, **dadurch gekennzeichnet, dass** die wärmenden Energiespeicher (9) die Form eines Kissens aufweisen.

10. Kiefer-Temperierband (1, 2) nach einem der vorhergehenden Ansprüche, **dadurch gekennzeichnet, dass** die wärmenden Energiespeicher (9) mit einem Naturprodukt, z.B. Dinkelkörner oder Rapssamen oder Reis oder Hülsenfrüchte oder Traubenkerne oder Kirschkerne oder Mischungen daraus, oder mit einem Gel gefüllt sind, wobei das Gel insbesondere aktivierbar ist zur Freisetzung der zuvor gespeicherten Energie.

11. Kiefer-Temperierband (1, 2) nach einem der vorhergehenden Ansprüche, **dadurch gekennzeichnet, dass** das Nacken-Unterkieferband (1) eine Größe von 103 x 30 cm aufweist.

12. Kiefer-Temperierband (1, 2) nach einem der vorhergehenden Ansprüche, **dadurch gekennzeichnet, dass** das Stirnband (2) eine Größe (angegeben als Länge mal Höhe in cm) von 30-60 x 6-12 cm aufweist, bevorzugt 45 x 8 cm.

## Claims

1. Jaw temperature-control strap (1, 2) for non-human mammals, namely for horses or other riding and work animals such as donkeys, camels and mules, comprising a basic cut adapted to the geometry of the horse or other riding and work animal, the basic cut having receiving devices (3, 4, 5) for accommodating warming energy storage devices (9), and the jaw temperature-control strap (1, 2) at least partially encompassing the lower jaw, preferably comprising the hyoid bone, and at least partially encompassing the upper jaw, the zygomatic arch, the sphenoid bone, the frontal bone and/or the parietal bone of the head and/or the neck of the horse or other riding and work animal, at least one receiving device (3, 4, 5) being arranged in/on the jaw temperature-control strap (1, 2) that encompasses the head, and the jaw temperature-control strap (1, 2) abutting on a masticatory muscle and/or a temporal muscle and/or an internal pterygoid muscle and/or an external pterygoid muscle of the skull over a surface facing the head and shared with the receiving device (3, 4, 5), the receiving devices being designed as pockets (3, 4, 5), the basic cut being designed at least in two parts and consisting of at least two strap elements (1, 2), with connection means (7, 8) being arranged on the strap elements for releasably connecting the strap elements, with one strap element, namely a forehead strap (2), being designed to extend over the mammal's forehead, and another strap element, namely a neck lower jaw strap (1), being designed to extend over the mammal's lower jaw and/or over the mammal's neck, the neck lower jaw strap (1) at least partially encompassing the neck, the lower jaw, preferably comprising the hyoid bone, and at least partially encompassing the upper jaw and the zygomatic arch, and the forehead strap (2) at least partially encompassing the sphenoid bone, the frontal bone and the jaw joint of the animal, the neck lower jaw strap (1) having a connection means (8) at its ends facing away from one another, by means of which the two ends can be connected, **characterised in that** the forehead strap (2) has a connection means (7, 8) at its ends and the neck jaw strap (1) has associated connection means (7, 8) so that the two straps can be connected.

2. Jaw temperature-control strap (1, 2) according to claim 1, **characterised in that** the receiving devices (3, 4, 5) are firmly integrated as pockets in the neck lower jaw strap (1) and in the forehead strap (2), the receiving devices (3, 4, 5) each preferably being designed to be divided into a plurality of pockets.

3. Jaw temperature-control strap (1, 2) according to either of the preceding claims, **characterised in that** the receiving devices (3, 4, 5) can be fixed to the neck lower jaw strap (1) and to the forehead strap (2) by means of a fastening means (10).

4. Jaw temperature-control strap (1, 2) according to any of the preceding claims, **characterised in that** a shape and size of the receiving device (3, 4, 5) can be selected on the basis of a predetermined muscle size.

5. Jaw temperature-control strap (1, 2) according to any of the preceding claims, **characterised in that** the connection means (7, 8) is designed as a hook-and-loop fastener or as a rivet fastener or as a strap/buckle fastener or as a click fastener or as a tape/loop fastener.

6. Jaw temperature-control strap (1, 2) according to any of the preceding claims, **characterised in that** it has at least one elastic material component, in particular the neck lower jaw strap (1) and the forehead strap (2) having at least one elastic material component, the material of the jaw temperature-control strap (1, 2), in particular of the neck lower jaw strap (1) and/or the forehead strap (2), preferably being made of a mixture of cotton and nylon or a mixture of nylon and polyester or exclusively of nylon.

7. Jaw temperature-control strap (1, 2) according to any of the preceding claims 1 to 6, **characterised in that** the neck lower jaw strap (1) and/or the forehead strap (2) is adapted to a predetermined size of the mammal's head.

8. Jaw temperature-control strap (1, 2) according to any of the preceding claims, **characterised in that** the neck lower jaw strap (1) has an elastic fixing strap (7), the end of which has an attachment which is located between the nasal bone and the cheekbone of the mammal.

9. Jaw temperature-control strap (1, 2) according to any of the preceding claims, **characterised in that** the warming energy storage devices (9) have the shape of a cushion.

10. Jaw temperature-control strap (1, 2) according to any of the preceding claims, **characterised in that** the warming energy storage devices (9) are filled with a natural product, e.g. spelt grains or rapeseed or rice or legumes or grape seeds or cherry stones or mixtures thereof, or with a gel, the gel in particular being activatable to release the previously stored energy.

11. Jaw temperature-control strap (1, 2) according to any of the preceding claims, **characterised in that** the neck lower jaw strap (1) has a size of 103 X 30 cm.

12. Jaw temperature-control strap (1, 2) according to any of the preceding claims, **characterised in that** the forehead strap (2) has a size (specified as length times height in cm) of 30-60 x 6-12 cm, preferably 45 x 8 cm.

## Revendications

1. Bande de tempérage de mâchoire (1, 2) pour mammifères non humains, à savoir pour des chevaux ou d'autres animaux de selle ou de trait tels les ânes, les chameaux ou les mulets, comprenant une pièce découpée de base adaptée à la géométrie du cheval ou d'un autre animal de selle ou de trait, la pièce découpée de base présentant des dispositifs de logement (3, 4, 5) pour recevoir des accumulateurs d'énergie chauffants (9), la bande de tempérage de mâchoire (1, 2) entourant en partie au moins la mâchoire inférieure, de préférence avec l'os hyoïde, la mâchoire supérieure, l'arcade zygomatique, l'os sphénoïde, l'os frontal et/ou l'os pariétal de la tête et/ou de la nuque du cheval ou d'un autre animal de selle ou de trait, un dispositif de logement (3, 4, 5) au moins étant placé dans ou contre la bande de tempérage de mâchoire (1, 2) entourant la tête et la bande de tempérage de mâchoire (1, 2) étant, par une surface commune au dispositif de logement (3, 4, 5) et orientée vers la tête, contiguë à un muscle masséter et/ou à un muscle temporal et/ou à un muscle ptérygoïdien médial et/ou à un muscle ptérygoïdien latéral du crâne, les dispositifs de logement étant réalisés sous forme de poches (3, 4, 5), la pièce découpée de base étant réalisée en au moins deux parties et comportant au moins deux éléments de bande (1, 2), des moyens d'assemblage (7, 8) étant disposés sur les éléments de bande pour les assembler de façon amovible, un élément de bande, à savoir une bande frontale (2), étant conçue pour s'étendre sur le front du mammifère et un autre élément de bande, à savoir une bande cervico-maxillaire (1), étant conçue pour s'étendre sur la mâchoire inférieure et/ou sur la nuque du mammifère, la bande cervico-maxillaire (1) entourant en partie au moins la nuque, la mâchoire inférieure, de préférence avec l'os hyoïde, la mâchoire supérieure et l'arcade zygomatique, et la bande frontale (2) entourant en partie au moins l'os sphénoïde, l'os frontal et l'articulation temporo-mandibulaire de l'animal, la bande cervico-maxillaire (1) présentant à ses extrémités opposées un moyen d'assemblage (8) permettant de relier les deux extrémités, **caractérisée en ce que** la bande frontale (2) ainsi que la bande cervico-maxillaire (1) présentent respectivement à leurs extrémités des moyens d'assemblage (7, 8) associés permettant de relier les deux bandes.

2. Bande de tempérage de mâchoire (1, 2) selon la revendication 1, **caractérisée en ce que** les dispositifs de logement (3, 4, 5) sont intégrés de manière fixe à la bande cervico-maxillaire (1) ainsi qu'à la bande frontale (2) sous forme de poches, les dispositifs de logement (3, 4, 5) étant de préférence respectivement subdivisées en plusieurs poches.

3. Bande de tempérage de mâchoire (1, 2) selon l'une des revendications précédentes, **caractérisée en ce que** les dispositifs de logement (3, 4, 5) peuvent être fixés à la bande cervico-maxillaire (1) ainsi qu'à la bande frontale (2) à l'aide d'un moyen de fixation (10).

4. Bande de tempérage de mâchoire (1, 2) selon l'une des revendications précédentes, **caractérisée en ce qu'**une forme et une taille du dispositif de logement (3, 4, 5) peuvent être choisies en fonction d'une taille de muscle déterminée.

5. Bande de tempérage de mâchoire (1, 2) selon l'une des revendications précédentes, **caractérisée en ce que** le moyen d'assemblage (7, 8) est réalisé sous forme de fermeture autoagrippante ou de fermoir à rivet ou de fermeture par sangle et boucle ou de fermoir à cliquet ou de fermeture par cordon et boucle.

6. Bande de tempérage de mâchoire (1, 2) selon l'une des revendications précédentes, **caractérisée en ce qu'**elle comporte au moins une partie en matériau élastique, la bande cervico-maxillaire (1) et la bande frontale (2) comportant notamment au moins une partie en matériau élastique, le matériau de la bande de tempérage de mâchoire (1, 2), notamment de la bande cervico-maxillaire (1) et/ou de la bande frontale (2), étant de préférence constitué d'un mélange de coton et de nylon ou d'un mélange de nylon et de polyester ou exclusivement de nylon.

7. Bande de tempérage de mâchoire (1, 2) selon l'une des revendications 1 à 6 précédentes, **caractérisée en ce que** la bande cervico-maxillaire (1) et/ou la bande frontale (2) sont adaptées à la taille de la tête du mammifère.

8. Bande de tempérage de mâchoire (1, 2) selon l'une des revendications précédentes, **caractérisée en ce que** la bande cervico-maxillaire (1) présente une bande de fixation élastique (7) dont l'extrémité est munie d'une fixation placée entre l'os nasal et l'os malaire du mammifère.

9. Bande de tempérage de mâchoire (1, 2) selon l'une des revendications précédentes, **caractérisée en ce que** les accumulateurs d'énergie chauffants (9) a la forme d'un coussin.

10. Bande de tempérage de mâchoire (1, 2) selon l'une des revendications précédentes, **caractérisée en ce que** les accumulateurs d'énergie chauffants (9) sont remplis d'un produit naturel, par ex. des graines d'épeautre ou de colza ou du riz, des légumes secs, des graines de raisin ou des noyaux de cerises ou encore d'un mélange de ces produits, ou d'un gel, le gel pouvant notamment être activé pour libérer l'énergie qui y a été préalablement accumulée.

11. Bande de tempérage de mâchoire (1, 2) selon l'une des revendications précédentes, **caractérisée en ce que** la bande cervico-maxillaire (1) a une taille de 103 x 30 cm.

12. Bande de tempérage de mâchoire (1, 2) selon l'une des revendications précédentes, **caractérisée en ce que** la bande frontale (2) a une taille (indiquée sous la forme longueur fois hauteur en cm) de 30-60 x 6-12 cm, de préférence de 45 x 8 cm.
